(19) Europäisches Patentamt / European Patent Office / Office européen des brevets

(11) **EP 3 476 275 A1**

(12) **EUROPEAN PATENT APPLICATION**

(43) Date of publication:
01.05.2019 Bulletin 2019/18

(51) Int Cl.:
*A61B 5/00* (2006.01)     *A61B 5/0408* (2006.01)
*A61B 5/026* (2006.01)    *A61B 5/024* (2006.01)
*A61B 5/02* (2006.01)      *A61B 5/11* (2006.01)

(21) Application number: 17198539.3

(22) Date of filing: 26.10.2017

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(71) Applicant: **Koninklijke Philips N.V.
5656 AE Eindhoven (NL)**

(72) Inventors:
• **VALENTI, Giulio
5656 AE Eindhoven (NL)**
• **TEN KATE, Warner Rudolph Theophile
5656 AE Eindhoven (NL)**

(74) Representative: **de Haan, Poul Erik
Philips International B.V.
Philips Intellectual Property & Standards
High Tech Campus 5
5656 AE Eindhoven (NL)**

(54) **PHYSIOLOGICAL SIGNAL PROCESSING APPARATUS AND METHOD**

(57)     A method and system for analyzing two related physiological signals to determine a time difference between them. The fundamental frequency components are analyzed to determine a time difference. This approach is robust against varying and different wave shapes, providing a stable and accurate determination of a time difference.

FIG. 1

**Description**

FIELD OF THE INVENTION

This invention relates to the processing of physiological signals, for example for blood pressure monitoring.

BACKGROUND OF THE INVENTION

Most, if not all, physiological processes are periodic in nature, where the period itself exhibits some (non-linear) variation. Examples include heart rate, breathing rate and walking gait. These phenomena can be measured using sensors. Depending on the used sensing principle the sensor signals themselves may have different shapes, but the periodicity will remain.

For example, a beating heart generates a periodic electrical signal, normally detected with an ECG, a periodic acoustic signal, normally detected with a stethoscope at the chest, and a periodic pressure wave, normally detected with a stethoscope at the limbs to evaluate blood pressure. Walking generates many periodic acceleration signals, which differ depending on body locations, which differences depend also on the rigidity of the body. There are also periodic acoustic signals due to the interaction with the ground, periodic pressure signals at the floor when touching the ground etc.

There is an increasing demand for unobtrusive health sensing systems based on monitoring physiological signals. In particular, there is a shift from conventional hospital treatment towards unobtrusive vital signs sensor technologies, centered around the individual, to provide better information about the subject's general health.

Such vital signs monitoring systems help to reduce treatment costs by disease prevention and enhance the quality of life. They may provide improved physiological data for physicians to analyze when attempting to diagnose a subject's general health condition. Vital signs monitoring typically includes monitoring one or more of the following physical parameters: heart rate, blood pressure, respiratory rate and core body temperature.

This invention relates generally to processing of periodic physiological signals. However, for the purposes of explanation, one particular application to blood pressure monitoring is discussed in detail.

In the US about 30% of the adult population has a high blood pressure. Only about 52% of this population have their condition under control. Hypertension is a common health problem which has no obvious symptoms and may ultimately cause death, and is therefore often referred to as the "silent killer". Blood pressure generally rises with aging and the risk of becoming hypertensive in later life is considerable. About 66% of the people in age group 65-74 have a high blood pressure. Persistent hypertension is one of the key risk factors for strokes, heart failure and increased mortality.

The condition of the hypertensive patients can be improved by lifestyle changes, healthy dietary choices and medication. Particularly for high risk patients, continuous 24 hour blood pressure monitoring is very important and there is obviously a desire for systems which do not impede ordinary daily life activities.

There are two main classes of method to monitor blood pressure.

For invasive direct blood pressure monitoring, the gold standard is by catheterization. A strain gauge in fluid is placed in direct contact with blood at any artery site. This method is only used when accurate continuous blood pressure monitoring is required in dynamic (clinical) circumstances. It is most commonly used in intensive care medicine and anesthesia to monitor blood pressure in real time.

For indirect blood pressure monitoring, oscillometry is a popular automatic method to measure blood pressure. The method uses a cuff with integrated pressure sensor. However, these methods are not suitable for performance by the user.

More non-invasive methods to estimate blood pressure are based on pulse wave velocity (PWV). This technique is based on the fact that the velocity of the pressure pulse traveling through an artery is related to blood pressure. The PWV is derived from the pulse transit time (PTT). Usually the pulse transit time is estimated by: (i) sampling and filtering the waveforms, (ii) detecting beats in the waveforms, (iii) detecting features within the beats that serve as a reference point (pulse arrival moment) and (iv) calculating the PTT as the time delay between the features. Thus, measurement of PTT involves determining a time difference between two physiological signals.

The PWV is then obtained by: PWV = D/PTT, where D refers to the pulse travel distance. The PWV can be translated to a blood pressure estimate by means of a calibration step. Often the Moens-Korteweg equation is applied which describes the relationship between blood pressure and PWV. If the pulse travel distance D is stable during a measurement, the blood pressure can be directly inferred from PTT by means of a calibration function.

The PTT is measurement is one example where the time shift between different signals generated by the same event can provide important information. The method currently used to determine the time shift between different signals generated by the same phenomenon requires making physiological assumptions about the shape of the wave to detect identifiable features as mentioned above. This can be very sensitive to signal noise. The determination of the two time instants becomes even more cumbersome when the two signal shapes are different. It is hard to identify which part of either curve corresponds to the same phase.

WO 2017/147609 discloses a system for calculating the PTT by processing a pair of PPG or ECG signals. Time-domain shift methods and frequency-domain phase measurement methods are disclosed.

There remains a need for a method and system which can determine time differences between related periodic signals (in particular signals having the same frequency) in a more reliable and automated manner.

SUMMARY OF THE INVENTION

The invention is defined by the claims.

According to examples in accordance with an aspect of the invention, there is provided a method of analyzing two related physiological signals to determine a time difference between them, comprising:

> receiving a first periodic physiological signal;
> receiving a second periodic physiological signal induced by the same physiological process as the first periodic physiological signal;
> identifying a fundamental frequency component of the first and second signals; and
> determining a time difference between the fundamental frequency components based on a time-domain time difference analysis or a frequency-domain group delay analysis, only of the fundamental frequency components.

This method enables automated detection of a time shift between two signals, based on identification and analysis of their fundamental frequency components and their associated phase relationship. The physiological signals are signals generated by the same phenomenon with a single characteristic frequency (at a given point in time) and hence the same period as each other. The method is robust against varying and different wave-shapes, in this way providing a more stable and accurate determination of the time difference.

The fundamental frequency is for example obtained from a spectral analysis such as a Fourier Transform applied to each signal, and identification of the peak in the transform.

The signal processing maintains the phase of the fundamental frequency component so that relative timing between the two fundamental frequency components is preserved. The time difference is determined based on analysis only of the fundamental frequency components (for both the frequency-domain approach and for the time-domain approach). By this is meant that the fundamental frequency components are extracted for analysis or else the signal processing takes place only at the frequency of the fundamental frequency component. In this way, timing is obtained based on signals which have corresponding time-domain characteristics (shape) or frequency-domain characteristics, so that reliable time delay calculation becomes possible.

The signal processing implements an effective normalization of the two signals based on their fundamental frequency components.

The first and second periodic physiological signals may both comprise:

> a PPG sensor signal; or
> an ECG signal; or
> a pressure sensor signal; or
> an acceleration sensor signal.

The signals may thus be of the same type and hence with the same basic frequency characteristics, but a time difference between the signals is of interest. This is for example possible when the first and second periodic physiological signals are received from different body locations.

The first and second periodic physiological signals may instead comprise different signals from the set of:

> a PPG sensor signal;
> an ECG signal;
> a pressure sensor signal; and
> an acceleration sensor signal.

In this case, a time delay between signals which have different shape may be of interest. This is for example possible when the first and second periodic physiological signals are received from the same body location. The different signals may have a different time response to a stimulus, in particular the signal shape (wave form) can be different, and the time difference is again of interest. By using on the fundamental frequency components for analysis, shape normalization takes place.

In a first set of examples, the method comprises extracting the identified fundamental frequency component. In this way, a new signal is generated which represents the original periodic physiological signals but which a much more easily identifiable phase. The extraction maintains the phase of the fundamental frequency components.

Extracting the fundamental frequency for example comprises applying a band pass filter in the frequency-domain.

The time difference may then be determined by aligning the band pass filtered components in the time-domain and determining a time shift needed for the alignment. Aligning the fundamental frequency components for example comprises maximizing a cross correlation between the fundamental frequency components.

By extracting the fundamental frequency component, for example with a band pass filter, the signals are processed such that they again have similar (sinusoidal) shapes. By having similar shapes the (cross) correlation will be larger at the correct time lag, while being low at other relative timing values.

This provides a time-domain approach by which the processing of the signals based on different candidate time delays results in the actual time difference between the fundamental frequency components being found.

In a second set of examples, determining the time difference comprises:

obtaining a frequency spectrum for the first and second periodic physiological signals; and:

determining a group delay time difference from the phase in the frequency spectrum in the vicinity of the fundamental frequency; or
combining the frequency spectra for the first and second periodic physiological signals and determining a group delay of the combined frequency spectrum.

This provides a frequency based analysis. The group delay is indicative of the rate of change of phase with respect to frequency. At the fundamental frequency, the group delay difference represents a time lag.

The method may be used for determining the pulse transit time, and for determining a blood pressure from the pulse transit time. In this way, the method enables non-invasive and indirect measurement of blood pressure by accurately and automatically determining the pulse transit time between two signals.

The method may be implemented at least in part in software.

The invention also provides a system for analyzing two related physiological signals to determine a time difference between them, comprising:

an input for receiving a first periodic physiological signal and a second periodic physiological signal induced by the same physiological process as the first periodic physiological signal;
a controller, which is adapted to:

identify a fundamental frequency component of the first and second signals; and
determine a time difference between the fundamental frequency components based on a time-domain time difference analysis or a frequency-domain group delay analysis, only of the fundamental frequency components. Identifying the fundamental frequency component for example involves finding the largest component for example based on the magnitude of the FFT spectrum. There are other options, such as the use of the discrete cosine transform.

The fundamental may instead be identified in the time-domain, by using a peak detector, determining the time between peaks, and determining the mean (or median). The inverse of the peak period time corresponds to the fundamental frequency.

The system may further comprise first and second physiological sensors, wherein:

the first and second physiological sensors both comprise a PPG sensor, an ECG sensor, a pressure sensor or an acceleration sensor; or
the first and second periodic physiological signals comprise different sensor from the set of a PPG sensor, an ECG sensor, a pressure sensor and an acceleration sensor.

In one set of examples, the controller comprises:

a band pass filter for extracting the identified fundamental frequency component; and
a cross correlator for aligning the fundamental frequency components thereby to find a time difference for which a maximized cross correlation is found.

This provides a time-domain analysis.

In another set of examples, the controller comprises:

a frequency analyzer for analyzing the first and second periodic physiological signals; and
group delay unit for finding a group delay from the phase in the frequency spectrum in the vicinity of the fundamental frequency and for determining a time difference between the group delays.

This provides a frequency-domain analysis.

BRIEF DESCRIPTION OF THE DRAWINGS

Examples of the invention will now be described in detail with reference to the accompanying drawings, in which:

Figure 1 shows a schematic example of a system for determining a time difference between physiological signals;
Figure 2 shows the units of a controller for a first processing approach;
Figure 3 shows the processing steps carried out by the controller of Figure 2
Figure 4 shows the units of a controller for a second processing approach;
Figure 5 shows the processing steps carried out by the controller of Figure 4; and
Figure 6 shows a method for determining a time difference between physiological signals.

DETAILED DESCRIPTION OF THE EMBODIMENTS

The invention will be described with reference to the Figures.
It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.
The invention provides a method and system for analyzing two related physiological signals to determine a time difference between them. The fundamental frequency components are analyzed to determine a time difference. This approach is robust against varying and different wave shapes, providing a stable and accurate determination of a time difference.

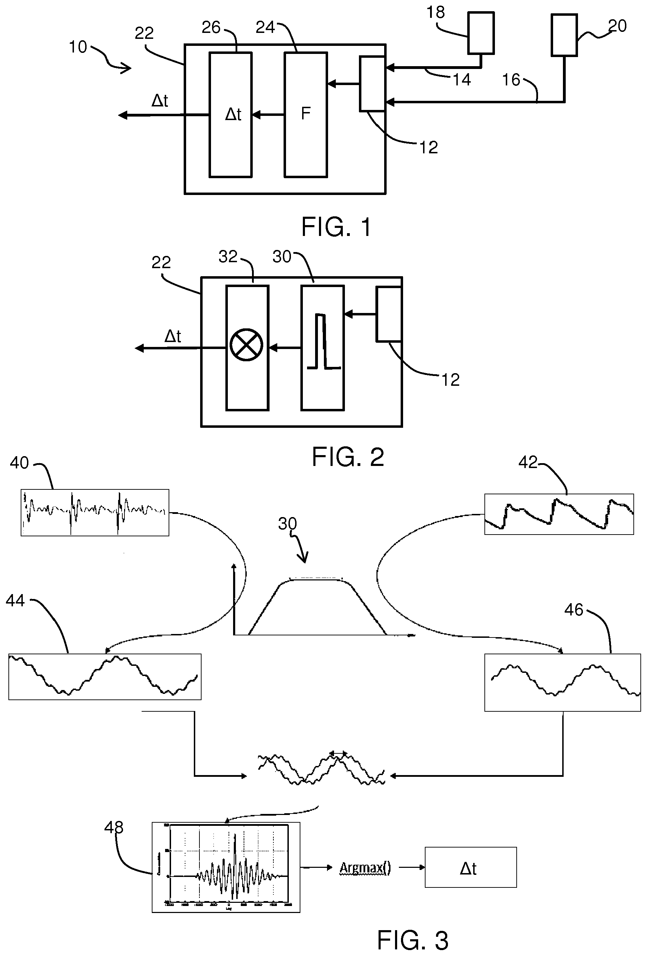

Figure 1 shows the system 10 in simplified schematic form. It comprises an input 12 for receiving a first periodic physiological signal 14 and a second periodic physiological signal 16 having the same period as the first periodic physiological signal. The first periodic signal is generated by a first physiological sensor 18 and the second periodic signal 16 is generated by a second physiological sensor 20.
The first and second physiological sensors may each comprise a PPG sensor, an ECG sensor, a pressure sensor or an acceleration sensor. Other sensors are also possible. The two sensors may be of the same type or they may be of different type. However, in all cases, the two signals have the same fundamental (dominant) frequency component, typically because this frequency component is induced by a common trigger, such as the heartbeat, or the respiration cycle, or walking. The invention is of interest when the relative timing of two signals, i.e. their phase difference, conveys useful information. One example is for the determination of pulse transit time, where the arrival of signals (having timing originally linked to the heartbeat) at different parts of the body enables physiological information to be derived. There are however other examples. For example, different signals may be measured at the same point, and time differences between those signals may provide information of relevance.
The system comprises a controller 22, which has a Fourier Transform unit 24 for identifying a fundamental frequency component of the first and second signals and a time calculation unit 26 which determines a time difference between the fundamental frequency components.
Identifying the fundamental frequency component for example involves finding the largest magnitude component of the FFT spectrum. However, there are other options, such as the use of the discrete cosine transform.
The fundamental frequency may instead be identified in the time-domain, by using a peak detector, determining the time between peaks, and determining the mean (or median). The inverse of the peak period time corresponds to the fundamental frequency. Since both signals have the same fundamental frequency, the analysis of both signals may be used to refine the estimate of the fundamental frequency. Instead of using peak detection, another characteristic such as like valley detection or or zero-crossing may be used.
There are different ways to determine the time difference.
Figure 2 shows a first example of an implementation based on a time-domain analysis. The controller 22 comprises the Fourier Transform unit 24 (e.g. a Fast Fourier Transform unit), a band pass filter 30 for extracting the identified fundamental frequency component and a cross correlator 32 for aligning the fundamental frequency components thereby to find a time difference for which a maximized cross correlation is found.

The band pass filter is centered at the fundamental frequency to remove noise and signal specific wavelengths, but it keeps the periodicity related to the underlying phenomenon. The resulting two near-sinusoidal curves are aligned by maximizing the cross correlation of the two filtered signals for varying delay. The time difference is the smallest delay (lag) at which the cross correlation maximizes.

Figure 3 shows this processing approach graphically.

The first physiological signal is shown as pane 40 and the second physiological signal is shown as pane 42. They are both processed by the band pass filter 30 to result in quasi-sinusoidal signals 44, 46.

The cross correlation signal 48 is then obtained and the maximum amplitude ("Argmax") corresponds to the time delay $\Delta$t.

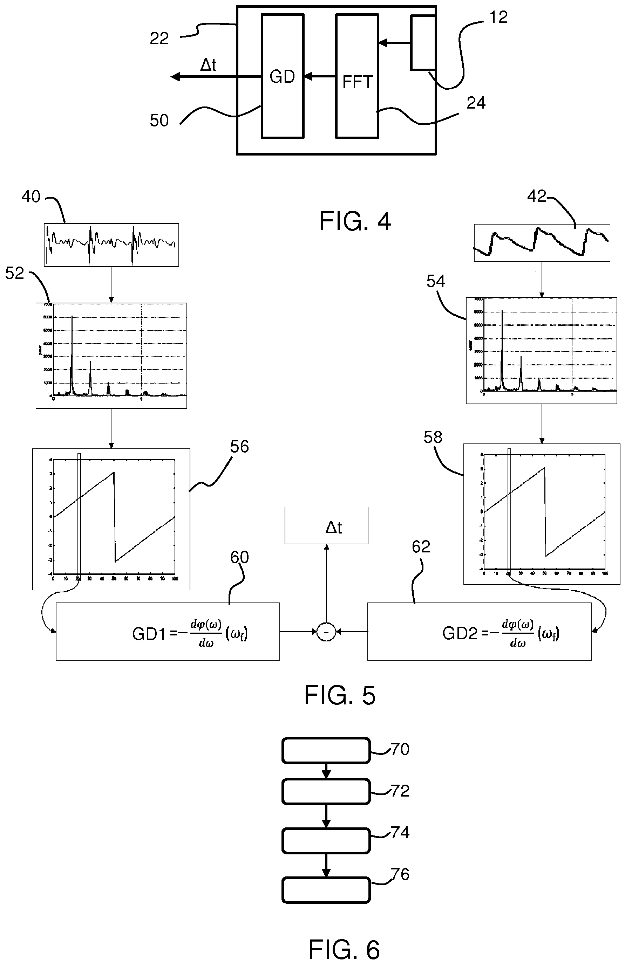

Figure 4 shows a second example of an implementation based on a frequency-domain analysis. The controller 22 comprises the Fourier Transform unit 24 (e.g. a Fast Fourier Transform unit), and a group delay unit 50. The Fourier Transform unit 24 functions as a frequency analyzer for analyzing the first and second periodic physiological signals. The group delay unit is for finding a group delay from the phase in the frequency spectrum in the vicinity of the fundamental frequency. The time delay is derived from the difference between the group delays.

The group delay, is the (negative) derivative of phase (lag) by frequency.

For example, it is assumed that a sinusoidal signal $y = \sin(\omega t+p)$ is delayed to $\sin(\omega(t-d)+p) = \sin(\omega t+(p-\omega d))$.

The phase lag is equal to $-\omega d$ and the group delay (the differential with respect to $\omega$) is equal to $-d$ (compared to zero for the non-delayed signal). Thus, the difference in group delay corresponds to the time difference.

Figure 5 shows this processing approach graphically.

The first physiological signal is again shown as pane 40 and the second physiological signal is shown as pane 42. They are both converted to frequency spectra to result in the signals 52, 54.

The phase is analyzed for the frequency centered at the identified fundamental frequency as represented by panes 56, 58.

The corresponding group delays GD1 60 and GD2 62 are obtained and the difference corresponds to the time delay.

Note that the group delay calculation is essentially a differentiation. The differentiation and the time difference calculation may be carried out in either order. Thus, this approach generally involves determining a group delay time difference from the phase in the frequency spectrum in the vicinity of the fundamental frequency. It may involve a phase difference calculation followed by a group delay calculation (the phase spectrum, after differentiation by frequency, becomes a group delay time value) or a group delay calculation followed by a time difference calculation (as explained fully above).

As an alternative, the two FFT signals can be first multiplied in the complex domain, and the group delay of the product is determined. This group delay is again the time difference of interest. Thus, in this case the frequency spectra for the first and second periodic physiological signals are first combined (multiplied) and the group delay of the combined frequency spectrum is then obtained.

In the complex frequency domain, the components may be expressed as Aexp(p), where A is the magnitude and p the phase (both dependent on frequency). Multiplying (with one complex conjugate) leads to subtracting phases:

$$[A_0 \exp(p_0)] \, . \, [\, A_1 . \exp(p_1) \,]^* = A_0 \exp(p_0) . A_1 \exp(-p_1) = A_0 A_1 . \exp(p_0 - p_1)$$

Figure 6 shows a method of analyzing two related physiological signals to determine a time difference between them, comprising:

in step 70, receiving a first periodic physiological signal;
in step 72, receiving a second periodic physiological signal having the same period as the first periodic physiological signal (because they are induced by the same physiological process);
in step 74 identifying a fundamental frequency component of the first and second signals; and
in step 76 determining a time difference between the fundamental frequency components.

This method enables automated detection of a time shift between two signals, based on identification and analysis of their fundamental frequency components and their associated phase relationship.

As mentioned above, one area of interest for the invention is in the monitoring of blood pressure. Specifically, signals related to heart rate can be recorded and compared with the system above. These signals may be of any type, such as PPG signals, ECG signals or pressure sensor signals. The pulse transit time is output as the time difference, and this can be used to monitor variations in blood pressure. A relative decrease in pulse transit time is associated with an increase in blood pressure. This is due to increased wave velocity due to the higher blood pressure. When the measuring locations are given, and thus the distance between measuring locations is known, the exact wave velocity can be calculated and thus an estimation of the absolute blood pressure is also possible.

The invention may also be used to define mechanical compliance of a system. For example, the system may be used to measure the rigidity of the human body during walking. Specifically, when the method is used to compare signals from different body locations (for example the neck and hip), the resulting time lag will be a function of the rigidity of the part of the system between the locations (in the example, the rigidity of the trunk). It is known that increased body rigidity is associated with increased fall risk so the system may be used as a monitoring system for vulnerable subjects. Together with fall risk monitoring, body rigidity could be used by a home monitoring system to monitor people with specific diseases related to posture. For example, people with Parkinson's disease show increased posture rigidity with the progression of the disease.

The time shift between two different types of signal measured at the same site can also give information about the originating event. For example, the delay between an ECG signal and the mechanical pulse auscultated at the chest indicates the mechanical answer time of the heart to the electrical stimulus given by a sinoatrial node. An increased delay indicates a decreased contractility of the heart tissues, which might lead to heart failure.

This method could also be used the opposite way: when the wearing location of one of the measuring sites is unknown, the time alignment could give indications about the distance of the other measuring site, and thus estimate the actual location. This method could be used to monitor changes in the wearing location. Changes in the time alignment over time might indicate a shift in the wearing location (example an arm/chest band that slips, or a pendant that lays in different positions).

Another example of a possible application of the invention is the comparison of breathing signals (for example by plethysmography) with the periodic variations in heart rate (for example by an optical heart rate monitor). The resulting delay can give an indication of how quickly the nervous system is capable of adapting heart rate to the internal pressure induced by breathing, and therefore give an estimation of the responsiveness of the nervous system.

As discussed above, a controller performs the data processing. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor or controller.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

**Claims**

**1.** A method of analyzing two related physiological signals to determine a time difference between them, comprising:

> (70) receiving a first periodic physiological signal (14);
> (72) receiving a second periodic physiological signal (16) induced by the same physiological process as the first periodic physiological signal;
> (74) identifying a fundamental frequency component of the first and second signals; and
> (76) determining a time difference between the fundamental frequency components based on a time-domain time difference analysis or a frequency-domain group delay analysis, only of the fundamental frequency components.

2. A method as claimed in claim 1, wherein the first and second periodic physiological signals (14, 16) both comprise:

   a PPG sensor signal; or
   an ECG signal; or
   a pressure sensor signal; or
   an acceleration sensor signal.

3. A method as claimed in claim 2, comprising receiving the first and second periodic physiological signals (14, 16) from different body locations.

4. A method as claimed in claim 1, wherein the first and second periodic physiological signals (14, 16) comprise different signals from the set of:

   a PPG sensor signal;
   an ECG signal;
   a pressure sensor signal; and
   an acceleration sensor signal.

5. A method as claimed in claim 4, comprising receiving the first and second periodic physiological signals (14, 16) from the same body location.

6. A method as claimed in any preceding claim, comprising extracting the identified fundamental frequency component.

7. A method as claimed in claim 6, wherein:

   extracting a fundamental frequency comprises applying a band pass filter (30) in the frequency-domain;
   determining the time difference comprises aligning the band pass filtered components in the time-domain and determining a time shift needed for the alignment.

8. A method as claimed in claim 7, wherein aligning the fundamental frequency components comprises maximizing a cross correlation between the fundamental frequency components.

9. A method as claimed in any one of claims 1 to 5, wherein determining the time difference comprises:

   obtaining a frequency spectrum for the first and second periodic physiological signals; and:

      determining a group delay time difference from the phase in the frequency spectrum in the vicinity of the fundamental frequency; or
      combining the frequency spectra for the first and second periodic physiological signals and determining a group delay of the combined frequency spectrum.

10. A method as claimed in any preceding claim for determining the pulse transit time, and determining a blood pressure from the pulse transit time.

11. A computer program comprising computer program code means which is adapted, when said program is run on a computer, to implement the method of any preceding claim.

12. A system for analyzing two related physiological signals to determine a time difference between them, comprising:

   an input (12) for receiving a first periodic physiological signal (14) and a second periodic physiological signal (16) induced by the same physiological process as the first periodic physiological signal;
   a controller (22), which is adapted to:

      identify a fundamental frequency component of the first and second signals; and
      determine a time difference between the fundamental frequency components based on a time-domain time difference analysis or a frequency-domain group delay analysis, only of the fundamental frequency components.

**13.** A system as claimed in claim 12, further comprising first and second physiological sensors (18, 20), wherein:

the first and second physiological sensors both comprise a PPG sensor, an ECG sensor, a pressure sensor or an acceleration sensor; or

the first and second periodic physiological signals comprise different sensor from the set of a PPG sensor, an ECG sensor, a pressure sensor and an acceleration sensor.

**14.** A system as claimed in claim 12 or 13, wherein the controller (22) comprises:

a band pass filter (30) for extracting the identified fundamental frequency component; and

a cross correlator (32) for aligning the fundamental frequency components thereby to find a time difference for which a maximized cross correlation is found.

**15.** A system as claimed in claim 12 or 13, wherein the controller (22) comprises:

a frequency analyzer (24) for analyzing the first and second periodic physiological signals; and

group delay unit (50) for finding a group delay from the phase in the frequency spectrum in the vicinity of the fundamental frequency and for determining a time difference between the group delays.

FIG. 1

FIG. 2

FIG. 3

**FIG. 4**

$$GD1 = -\frac{d\varphi(\omega)}{d\omega}(\omega_f)$$

$$GD2 = -\frac{d\varphi(\omega)}{d\omega}(\omega_f)$$

$\Delta t$

**FIG. 5**

**FIG. 6**

Europäisches
Patentamt

European
Patent Office

Office européen
des brevets

# EUROPEAN SEARCH REPORT

Application Number

EP 17 19 8539

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2017/142668 A1 (QUALCOMM INC [US]) 24 August 2017 (2017-08-24) <br> * page 9, line 30 * <br> * page 10, line 6 * <br> * page 13, line 12 * <br> * page 14, line 15 * <br> * page 15, lines 9, 10, 14 * <br> * page 16, lines 7, 8, 12 * <br> * page 17, lines 13, 17 * <br> * page 21, line 10 * <br> * page 27, lines 19, 23 * <br> * page 28, lines 2, 3 * <br> * page 48, lines 28, 29, 31 * <br> * figure 4 * | 1-15 | INV. <br> A61B5/00 <br> A61B5/0408 <br> A61B5/026 <br> A61B5/024 <br> A61B5/02 <br><br> ADD. <br> A61B5/11 |
| A | US 2009/156948 A1 (SHIMIZU HIDEKI [JP] ET AL) 18 June 2009 (2009-06-18) <br> * paragraphs [0159], [0170] * <br> * figures 4-9 * | 1,2,6, 11,12,14 | |
| A | BOREOM LEE ET AL: "Adaptive comb filtering for motion artifact reduction from PPG with a structure of adaptive lattice IIR notch filter", ENGINEERING IN MEDICINE AND BIOLOGY SOCIETY,EMBC, 2011 ANNUAL INTERNATIONAL CONFERENCE OF THE IEEE, IEEE, 30 August 2011 (2011-08-30), pages 7937-7940, XP032320521, DOI: 10.1109/IEMBS.2011.6091957 ISBN: 978-1-4244-4121-1 <br> * page 7937, left-hand column, line 1 * <br> * page 7939, left-hand column, line 10 - line 15 * | 1,6,11, 12,14 | TECHNICAL FIELDS SEARCHED (IPC) <br><br> A61B |
| A | US 2013/109982 A1 (SATO HIRONORI [JP] ET AL) 2 May 2013 (2013-05-02) <br> * paragraph [0056] - paragraph [0059] * | 9,15 | |

The present search report has been drawn up for all claims

| Place of search | Date of completion of the search | Examiner |
|---|---|---|
| The Hague | 4 April 2018 | Meyer, Wolfgang |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 17 19 8539

This annex lists the patent family members relating to the patent documents cited in the above-mentioned European search report.
The members are as contained in the European Patent Office EDP file on
The European Patent Office is in no way liable for these particulars which are merely given for the purpose of information.

04-04-2018

| Patent document cited in search report | Publication date | Patent family member(s) | Publication date |
|---|---|---|---|
| WO 2017142668 A1 | 24-08-2017 | US 2017238817 A1<br>WO 2017142668 A1 | 24-08-2017<br>24-08-2017 |
| US 2009156948 A1 | 18-06-2009 | CN 101272731 A<br>JP 4657300 B2<br>JP WO2007037100 A1<br>US 2009156948 A1<br>WO 2007037100 A1 | 24-09-2008<br>23-03-2011<br>02-04-2009<br>18-06-2009<br>05-04-2007 |
| US 2013109982 A1 | 02-05-2013 | CN 103002798 A<br>DE 112011102420 T5<br>JP 5615073 B2<br>JP 2012024156 A<br>US 2013109982 A1<br>WO 2012011463 A1 | 27-03-2013<br>16-05-2013<br>29-10-2014<br>09-02-2012<br>02-05-2013<br>26-01-2012 |

EPO FORM P0459

For more details about this annex : see Official Journal of the European Patent Office, No. 12/82

## REFERENCES CITED IN THE DESCRIPTION

**Patent documents cited in the description**

- WO 2017147609 A